# EUROPEAN PATENT SPECIFICATION

(11) **EP 1 165 115 B1**
(45) Date of publication and mention of the grant of the patent: **28.05.2003**
(21) Application number: 00916545.7
(22) Date of filing: 20.03.2000
(51) Int. Cl.: A61K 38/17, A61K 38/18, A61K 39/395, A61P 9/00

(54) **MODULATION OF VASCULAR PERMEABILITY BY MEAN OF TIE2 RECEPTOR ACTIVATORS**
MODULIERUNG DER GEFÄSSEPERMEABILITÄT MITTELS TIE2 REZEPTORAKTIVATOREN
MODULATION DE LA PERMEABILITE VASCULAIRE AU MOYEN D'ACTIVATEURS DU RECEPTEUR TIE2

(30) Priority: 26.03.1999 US 126539 P; 05.04.1999 US 286033
(43) Date of publication of application: 02.01.2002
(73) Proprietor: REGENERON PHARMACEUTICALS, INC., Tarrytown, NY 10591-6707 (US); The Regents of the University of California, Oakland, CA 94607-5200 (US)
(72) Inventor: SURI, Chitra, Tarrytown, NY 10591 (US); YANCOPOULOS, George, D., Yorktown Heights, NY 10598 (US); THURSTON, Gavin, White Plains, NY 10603 (US); MCDONALD, Donald, San Francisco, CA 94118 (US)
(74) Representative: Duckworth, Timothy John
(86) International application number: US0007336
(87) International publication number: WO00057901

(56) References cited:
- WO-A-00/18804
- WO-A-98/07832
- US-A- 5 482 930
- PATENT ABSTRACTS OF JAPAN vol. 011, no. 273 (C-445), 4 September 1987 (1987-09-04) -& JP 62 077318 A (EISAI CO LTD), 9 April 1987 (1987-04-09)
- DATABASE MEDLINE [Online] US NATIONAL LIBRARY OF MEDICINE (NLM), BETHESDA, MD, US; DVORAK H F ET AL: "Vascular permeability factor/vascular endothelial growth factor and the significance of microvascular hyperpermeability in angiogenesis." retrieved from STN Database accession no. 1999109226 XP002143874 & CURRENT TOPICS IN MICROBIOLOGY AND IMMUNOLOGY, (1999) 237 97-132. REF: 171 ,
- MAISONPIERRE PETER C ET AL: "Angiopoietin-2, a natural antagonist for Tie2 that disrupts in vivo angiogenesis." SCIENCE (WASHINGTON D C), vol. 277, no. 5322, 1997, pages 55-60, XP002143872 ISSN: 0036-8075
- THURSTON GAVIN ET AL: "Angiopoietin-1 protects the adult vasculature against plasma leakage." NATURE MEDICINE, vol. 6, no. 4, April 2000 (2000-04), pages 460-463, XP002143873 ISSN: 1078-8956

## Description

### Field of the Invention

The invention generally relates to angiogenic factors and more particularly to the angiopoietin family of growth factors and to methods of decreasing or inhibiting vascular permeability and/or plasma leakage.

### Background of the invention

Plasma leakage, a key component of inflammation, occurs in a distinct subset of the microvessels. In particular, in most organs plasma leakage occurs specifically in the venules. Unlike arterioles and capillaries, venules become leaky in response to numerous inflammatory mediators including histamine, bradykinin, and serotonin.

One characteristic of inflammation is the plasma leakage that results from intercellular gaps that form in the endothelium of venules. Most experimental models of inflammation indicate that these intercellular gaps occur between the endothelial cells of postcapillary and collecting venules (Baluk, P., et al., Am. J. Pathol. 1998 152:1463-76). It has been shown that certain lectins may be used to reveal features of focal sites of plasma leakage, endothelial gaps, and finger-like processes at endothelial cell borders in inflamed venules (Thurston, G., et al., Am. J. Physiol, 1996, 271: H2547-62). In particular, plant lectins have been used to visualize morphological changes at endothelial cell borders in inflamed venules of, for example, the rat trachea. Lectins, such as concanavalin A and ricin, that bind focally to inflamed venules reveal regions of the subendothelial vessel wall exposed by gaps that correspond to sites of plasma leakage (Thurston, G., et al., Am J Physiol, 1996, 271: H2547-62).

The properties of the microvessels are dynamic. Chronic inflammatory diseases, for example, are associated with microvascular remodeling, including angiogenesis and microvessel enlargement. Microvessels can also remodel by acquiring abnormal phenotypic properties. In a murine model of chronic airway inflammation, we found that airway capillaries acquire properties of venules, including widened vessel diameter, increased immunoreactivity for von Willebrand factor, and increased immunoreactivity for P-selectin. In addition, these remodeled vessels leak in response to inflammatory mediators, whereas vessels in the same position in the airways of normal mice do not.

Certain substances have been shown to decrease or inhibit vascular permeability and/or plasma leakage. For example, mystixins are synthetic polypeptides that have been reported to inhibit plasma leakage without blocking endothelial gap formation (Baluk, P., et al., J. Pharmacol. Exp. Ther., 1998, 284: 693-9). Also, the beta 2-adrenergic receptor agonist formoterol reduces microvascular leakage by inhibiting endothelial gap formation (Baluk, P. and McDonald, D.M., Am. J. Physiol., 1994, 266:L461-8).

What factors determine whether a vessel will acquire phenotypic features of venules? One apparent clue came from studies of angiopoietin-1 (Ang1), a ligand for the endothelial cell-specific receptor TIE2. In mice that transgenically overexpress Ang1 in the skin under the keratin-14 promoter (K14-Ang1 mice), microvessels in the position of capillaries have widened vessel diameter, immunoreactivity for P-selectin, and immunoreactivity for von Willebrand factor. Thus, these vessels have phenotypic features of venules.

Chronic inflammation is associated with blood vessel formation and enlargement and changes in vessel phenotype. In mice with chronic airway inflammation, strain-dependent differences to the same stimulus have been shown to result in either blood vessel proliferation or enlargement, depending on the host response (Thurston, G., et al., Am. J. Pathol., 1998, 153: 1099-112). Analyses of mouse embryos deficient in the TIE2 receptor illustrate its importance in angiogenesis, particularly for vascular network formation in endothelial cells. Sato, T.N., et al., Nature 376:70-74 (1995). In the mature vascular system, the TIEs could function in endothelial cell survival, maintenance and response to pathogenic influences.

It has been suggested that the TIE receptors play roles in endothelial cell determination, proliferation, differentiation and cell migration and patterning into vascular elements. The predominant expression of the TIE receptors in vascular endothelia suggests that the TIEs play a role in the development and maintenance of the vascular system. The TIE receptors are also expressed in primitive hematopoietic stem cells, B cells and a subset of megakaryocytic cells, thus suggesting the role of ligands which bind these receptors in early hematopoiesis, in the differentiation and/or proliferation of B cells, and in the megakaryocytic differentiation pathway. Iwama, et al. Biochem. Biophys. Research Communications 195:301-309 (1993); Hashiyama, et al. Blood 87:93-101 (1996); Batard, et al. Blood 87:2212-2220 (1996).

An angiogenic factor, which was originally called TIE2 ligand-1 (TL1) but is also referred to as angiopoietin-1 (Ang1), has been identified that signals through the TIE2 receptor and is essential for normal vascular development in the mouse. By homology screening, an Ang1 relative has been identified and called TIE2 ligand-2 (TL2) or angiopoietin-2 (Ang-2). Ang-2 is a naturally occurring antagonist for Ang1 and the TIE2 receptor. For a description of the cloning and sequencing of TL1 (Ang1) and TL2 (Ang-2) as well as for methods of making and uses thereof, reference is hereby made to PCT International Publication No. WO 96/11269 published 18 April 1996 and PCT International Publication No. WO 96/31598 published 10 October 1996 both in the name of Regeneron Pharmaceuticals, Inc.; and S. Davis, et al., Cell 87: 1161-1169 (1996) each of which is hereby incorporated by reference. Ang1* is a mutant form of Ang1 that comprises the N-terminal domain of Ang-2 fused to the coiled-coil domain and the fibrinogen domain of Ang1 and that has a Cys to Ser mutation at amino acid 245 (See PCT International Publication No. WO 98/05779 published 12 February 1998 in the name of Regeneron Pharmaceuticals, Inc.). Ang1* has been shown to be a potent agonist for the TIE2 receptor.

Including the above-described angiopoietins, Applicants have identified a family of several related angiogenic factors. These have been designated TIE2 ligand-1 (TL1) also referred to as angiopoietin-1 (Ang1); TIE2 ligand-2 (TL2) or angiopoietin-2 (Ang-2); Tie ligand-3 (TL3) or angiopoietin-3 (Ang3) and Tie ligand-4 (TL4) or angiopoietin-4 (Ang4). For descriptions of the structural and functional properties of these four related factors, reference is hereby made to the following publications : U.S. Patent No. 5,643,755, issued 7/1/97 to Davis, et al.; U.S. Patent No. 5,521,073, issued 5/28/96 to Davis, et al.; U.S. Patent No. 5,650,490, issued 7/22/97 to Davis, et al.; U.S. Patent No. 5,879,672, issued March 9, 1999; U.S. Patent No. 5,814,464, issued September 29, 1998; U.S. Patent No. 5,851,797, issued December 22, 1998; PCT International Application No. PCT/US95/12935, filed Oct. 6, 1995, published on April 18, 1996, with Publication No. WO 96/11269; and PCT International Application No. PCT/US96/04806, filed April 5, 1996, published on October 10, 1996, with Publication No. WO96/3159 both PCT applications in the name of Regeneron Pharmaceuticals, Inc.

The angiopoietins can be structurally divided into three domains: an N-terminal region lacking homology to any known structures; an alpha-helical-rich coil-coil segment similar to motifs found in many proteins that seem to promote multimerization; and a "fibrinogen-like domain" thus dubbed because it is distantly related by homology to a domain first found in fibrinogen but now noted to be in many other proteins (Davis, S. et al., (1996) Cell 87: 1161-1169). The fibrinogen-like domain represents the most conserved region of the angiopoietins, and recent studies indicate that it comprises the receptor-binding portion of the angiopoietins. In addition, all the information that determines whether an angiopoietin is an agonist or an antagonist of the TIE receptors appears to reside within the fibrinogen-like domain. For example, when chimeric molecules are made in which the fibrinogen-like domains of Ang1 and Ang-2 are swapped, agonistic or antagonistic abilities track with the fibrinogen-like domains. The N-terminal and coil-coil regions appear to serve mainly to multimerize the fibrinogen-like domains, which apparently must be clustered to be active. In fact, the N-terminal and coil-coil regions can be substituted with alternative motifs that allow clustering or multimerization. Thus, the activities of Ang1 and Ang-2 can be precisely mimicked by surrogates in which the fibrinogen-like domains (FD) of these factors are fused to the constant region of an antibody, resulting in FD-Fc fusions, which can then be clustered using secondary antibodies directed against the Fc. For a general description of the production and use of FD-Fc fusions, see International Publication Number WO 97/48804 published 24 December 1997. Using these techniques, one of skill in the art would be able to similarly make FD-Fc fusions using the fibrinogen-like domain of any angiopoietin family member. One practical advantage of such surrogates is that native angiopoietins can be difficult to produce recombinantly, while the surrogates can be more easily produced.

As described in C. Suri, et al., Cell, 1996, 87: 1171-1180, the absence of Ang1 causes severe vascular abnormalities in the developing mouse embryo. Ang1 and Ang-2 have been described as naturally occurring positive (agonist) and negative (antagonist) regulators of angiogenesis. Positive or negative regulation of TIE2 is likely to result in different outcomes depending on the combination of simultaneously acting angiogenic signals.

The angiopoietins and members of the vascular endothelial growth factor (VEGF) family are the only growth factors thought to be largely specific for vascular endothelial cells. Targeted gene inactivation studies in mice have shown that VEGF is necessary for the early stages of vascular development and that Ang1 is required for later stages of vascular remodeling. It has been reported that transgenic overexpression of Ang1 in the skin of mice produces larger, more numerous, and more highly branched vessels, however the characteristics of the resultant vessels are largely unknown (Suri, C., et al., Science, 1998, 282:468-471). The present invention is the result of Applicants' efforts to examine these vessels in greater detail by, among other studies, assaying for vessel permeability/plasma leakage.

### Summary of the Invention

The present invention provides use of a TIE-2 receptor activator (agonist) in the manufacture of a medicament for use in a method of decreasing or inhibiting plasma leakage (including vascular permeability) in a mammal.

By way of example, but not by way of limitation, the vascular permeability/plasma leakage may be produced by an inflammatory agent or by injury. In one embodiment of the invention, the mammal is a human. By vascular permeability, what is meant is any process that leads to leakage or extravasation of plasma including, but not limited to, increased endothelial permeability. See for example, McDonald, D.M., et al., Microcirculation 6: 7-22 (1999); Feng, D., et al., Microcirculation 6: 23-44 (1999); and Michel, C.C., and Neal, C.R., Microcirculation 6: 45-54 (1999).

The invention further provides for a use wherein the TIE2 receptor activator is Ang1, or a polypeptide comprising a fragment or derivative thereof capable of activating the TIE2 receptor.

The invention also provides for a use wherein the TIE2 receptor activator is an activating antibody, or a polypeptide comprising a fragment or derivative thereof capable of activating the TIE2 receptor.

The invention further provides for a use wherein the TIE2 receptor activator is a small molecule capable of activating the TIE2 receptor.

The invention also provides use of an anti-Ang2 neutralising antibody in the manufacture of a medicament for use in a method of decreasing or inhibiting plasma leakage (including vascular permeability) in a mammal. In one embodiment of this invention, the mammal is a human. The invention also provides for a use wherein the anti-Ang-2 neutralizing antibody is a monoclonal antibody, including a wholly human monoclonal antibody. The invention further provides for a use wherein the anti-Ang-2 neutralizing antibody is a polyclonal antiserum.

### Brief Description of the Figures

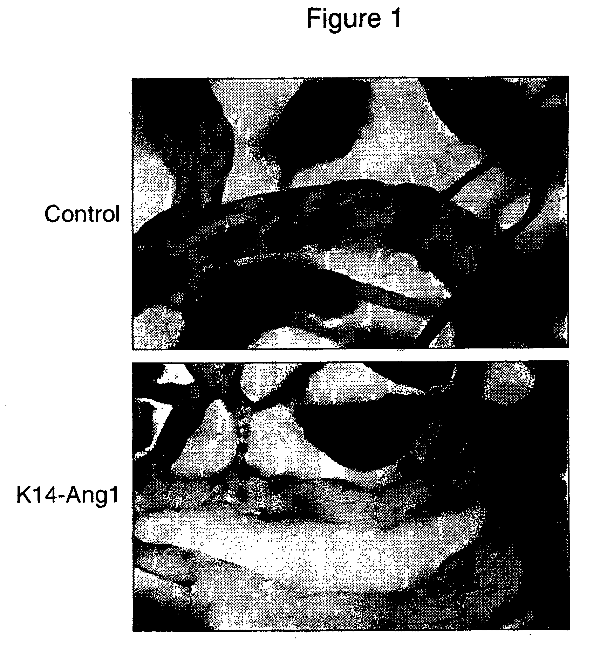

FIGURE 1 - Ricin Stain Showing Vessels in Ang1 Over-expressing Mice are resistant to Mustard Oil Induced Vascular Leakage - The microvasculature of mice over-expressing Ang1 in the skin (K14-Ang1 - bottom panel) and wild-type control FVB/N mice (control - top panel) was stained by perfusion of lectin (ricin) after treatment with the inflammatory agent mustard oil, then examined in whole mounts of the ear skin.

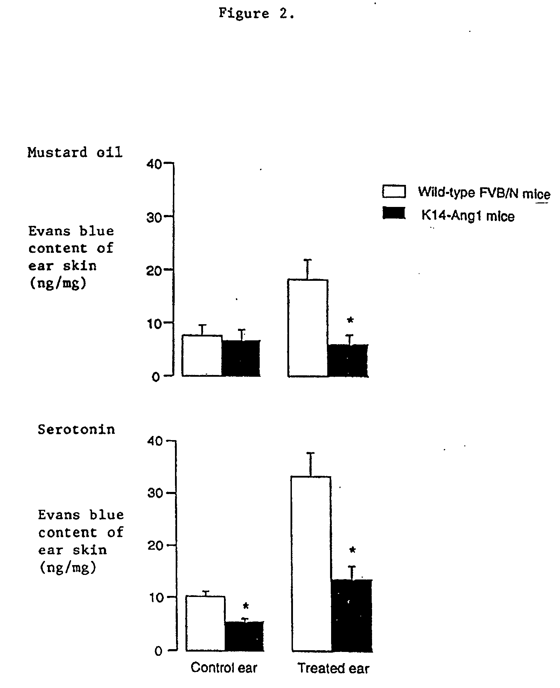

FIGURE 2 - Leakage From Ear Vessels of Wild-Type and K14-Ang1 Mice -Mustard oil (5%) was applied to surface of ear skin, control ear received no treatment. Serotonin (10 µg/ml) was injected dermally, control ear received injection of vehicle. Leakage was measured at 30 min after stimulus, n= 4 to 6 ears per group, values are mean ± SE. *Significantly different from corresponding wild=type value, P< 0.05, Bonferroni/Dunn test.

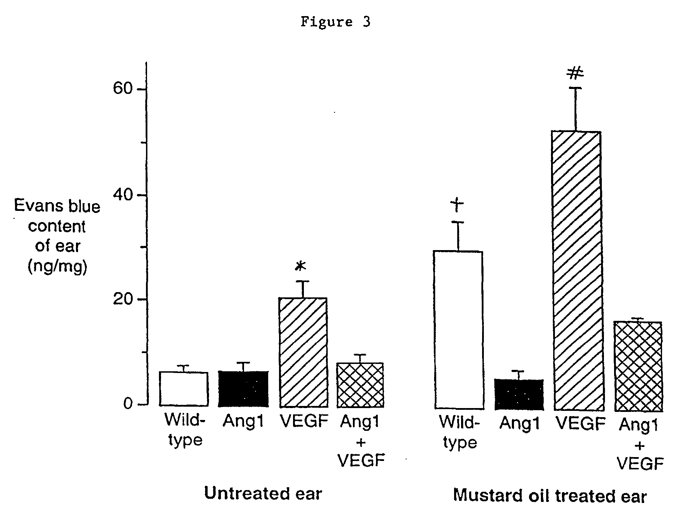

FIGURE 3 - Amount of extravasation of Evans blue in ears of wild-type, K14-Ang1, K14-VEGF, and K14-Ang1/VEGF mice 30 minutes after topical application of mustard oil. Asterisk marks significantly greater leakage in untreated ears of K14-VEGF mice than in untreated ears of wild-type, K14-Ang1, or K14-Ang1/VEGF mice. Cross marks significantly greater leakage in treated ears of wild-type mice than in treated ears of K14-Ang1 or K14-Ang1/VEGF mice. # marks significantly greater leakage in treated ears of K14-VEGF mice than in treated ears of K14-Ang1 or K14-Ang1/VEGF mice.

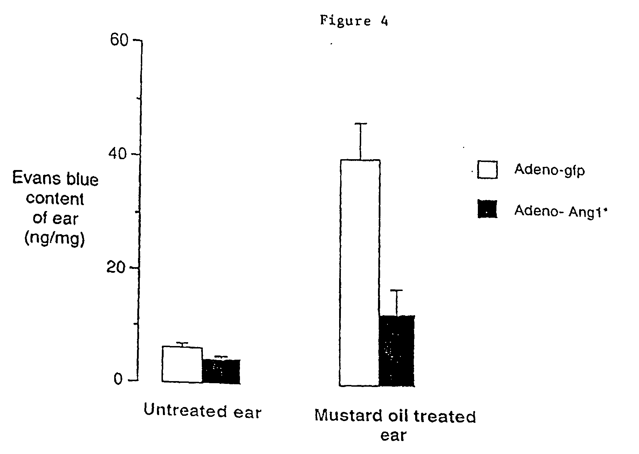

FIGURE 4 - Amount of extravasation of Evans blue in ears of wild-type mice injected intravenously with adenovirus expressing Ang1* or adenovirus expressing green fluorescent protein six days earlier, and then treated for 30 minutes by topical application of mustard oil.

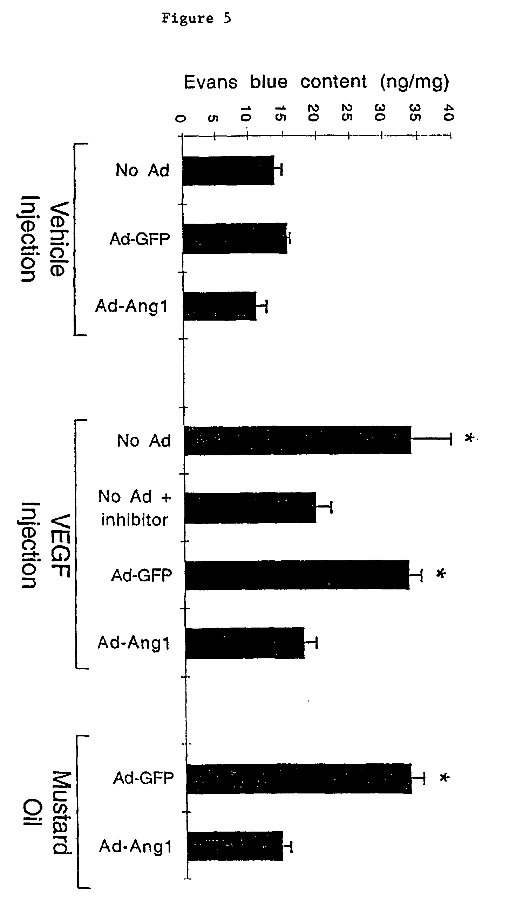

FIGURE 5 - Quantitation of tissue levels of Evans blue dye in ears from mice pre-treated systemically with either Ad-Ang1 or Ad-GFP, and 5-7 days later challenged with intradermal injections of VEGF protein or saline (sterile solution of 0.9% NaCI) control; mustard oil-treated ears serve as an additional control. Also shown is blockage of the VEGF effect using co-injection of a specific VEGF antagonist, i.e. a 10-fold molar excess of a soluble VEGFR1-Fc fusion protein.
*, Significantly greater leakage than in control and Ad-Ang1 treated mice.

### Detailed Description of the Invention

As described in greater detail below, Applicants have found that a TIE-2 receptor activator may be used to decrease or inhibit vascular permeability and/or plasma leakage in a mammal. By way of example, but not by way of limitation, the vascular permeability/plasma leakage may be produced by an inflammatory agent or by injury. In one embodiment of the invention, the mammal is a human.

The TIE2 receptor activator may be Ang1, or a polypeptide comprising a fragment or derivative thereof capable of activating the TIE2 receptor.

The TIE2 receptor activator may alternatively be an activating antibody, or a polypeptide comprising a fragment or derivative thereof capable of activating the TIE2 receptor.

The TIE2 receptor activator may, in a further alternative, be a small molecule capable of activating the TIE2 receptor.

An anti-Ang2 neutralising antibody may also be used to decrease or inhibit vascular permeability and/or plasma leakage in a mammal.

In one embodiment of this invention, the mammal is a human. The invention also provides for a use wherein the anti-Ang-2 neutralizing antibody is a monoclonal antibody, including a wholly human monoclonal antibody. The invention further provides for a use wherein the anti-Ang-2 neutralizing antibody is a polyclonal antiserum.

By way of example, but not limitation, the invention may be useful in treating clinical conditions that are characterized by plasma leakage/vascular permeability, edema or inflammation such as brain edema associated with injury, stroke or tumor; edema associated with inflammatory disorders such as psoriasis or arthritis, including rheumatoid arthritis; asthma; generalized edema associated with burns; ascites and pleural effusion associated with tumors, inflammation or trauma; chronic airway inflammation; capillary leak syndrome; sepsis; kidney disease associated with increased leakage of protein; and eye disorders such as age-related macular degeneration and diabetic retinopathy.

The compositions of the invention may be administered systemically or locally. Any appropriate mode of administration known in the art may be used, including, but not limited to, intravenous, intrathecal, intraarterial, intranasal, oral, subcutaneous, intraperitoneal, or by local injection or surgical implant. Sustained release formulations are also provided for.

For preparation of monoclonal antibodies, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in "Monoclonal Antibodies and Cancer Therapy," Alan R. Liss, Inc. pp. 77-96) and the like are within the scope of the present invention.

The monoclonal antibodies may be human monoclonal antibodies or chimeric human-mouse (or other species) monoclonal antibodies. Human monoclonal antibodies may be made by any of numerous techniques known in the art (e.g., Teng et al., 1983, Proc. Natl. Acad. Sci. U.S.A. 80:7308-7312; Kozbor et al., 1983, Immunology Today 4:72-79; Olsson et al., 1982, Meth. Enzymol. 92:3-16). Chimeric antibody molecules may be prepared containing a mouse antigen-binding domain with human constant regions (Morrison et al., 1984, Proc. Natl. Acad. Sci. U.S.A. 81:6851, Takeda et al., 1985, Nature 314:452). Wholly human monoclonal antibodies are also provided for and can be made as described in, for example, US Patent No. 5,939,598, issued August 17, 1999 and assigned to Abgenix, Inc.

Various procedures known in the art may be used for the production of polyclonal antibodies. For the production of Ang-2 inactivating antibodies, various host animals, including but not limited to rabbits, mice and rats can be immunized by injection with Ang-2, or a fragment or derivative thereof. For the production of TIE2 activating antibodies, various host animals, including but not limited to rabbits, mice and rats can be immunized by injection with TIE2 receptor extracellular domain, or a fragment or derivative thereof. Various adjuvants may be used to increase the immunological response, depending on the host species, and including but not limited to Freund's (complete and incomplete), mineral gels such as aluminum hydroxide, surface active substances such as lysolecithin, pluronic polyols, polyanions, polypeptides, oil emulsions, keyhole limpet hemocyanins, dinitrophenol, and potentially useful human adjuvants such as BCG (Bacille Calmette-Guerin) and Corynebacterium parvum.

A molecular clone of an antibody to a selected epitope can be prepared by known techniques. Recombinant DNA methodology (see e.g., Maniatis et al., 1982, Molecular Cloning, A Laboratory Manual, Cold Spring Harbor Laboratory, Cold Spring Harbor, New York) may be used to construct nucleic acid sequences which encode a monoclonal antibody molecule, or antigen binding region thereof.

The present invention provides for antibody molecules as well as fragments of such antibody molecules. Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent. Antibody molecules may be purified by known techniques, e.g., immunoabsorption or immunoaffinity chromatography, chromatographic methods such as HPLC (high performance liquid chromatography), or a combination thereof.

In alternative embodiments of the invention, a single chain Fv may be engineered. A single chain Fv (scFv) is a truncated Fab having only the V region of a heavy chain linked by a stretch of synthetic peptide to a V region of a light chain. See, for example, US Patent Nos. 5,565,332; 5,733,743; 5,837,242; 5,858,657; and 5,871,907 assigned to Cambridge Antibody Technology Limited.

The following examples are offered by way of illustration and not by way of limitation.

### Examples

### Example 1:

It was previously shown that transgenic overexpression of Ang1 in the skin of mice (K14-Ang1) as described by Suri, C., et al., 1998, Science 282: 468-471, produces larger, more numerous, and more highly branched vessels. The microvasculature of mice over-expressing Ang1 in the skin (K14-Ang1) and wild-type control FVB/N mice was then stained by perfusion of lectin, as generally described in Thurston, G. et al., 1998, Am. J. Pathol 153(4):1099-112, then examined in whole mounts of the ear skin as shown in Figure 1.

Plasma leakage from ear vessels was measured in some mice after topical application of the inflammatory agents mustard oil or serotonin (See Inoue, H, et al., 1997, Eur. J. Pharmacol. 333:231-40) with the tracer Evans blue (See Baluk, P., et al., 1997, Am. J. Physiol. 272:L155-170). Plasma leakage was measured using the tracer dye Evans blue. Evans blue (EM Sciences, Cherry Hill, NJ) 30 mg/kg in a volume of 100 µl was injected into one femoral vein of anesthetized mice, and 1 min later mustard oil or serotonin was administered to one ear. Mustard oil (Sigma, St. Louis, MO) was diluted to 5% in mineral oil and applied with a Q-tip to the dorsal and ventral surfaces of the skin of one ear, and nothing was done to the other ear (baseline control). Serotonin (Sigma) was made up at 0.22 mg/ml in sterile saline plus 0.005 N acetic acid (vehicle). Approximately 10 µl was injected intradermally into the dorsal ear skin, and a similar volume of vehicle was injected into the other ear (control). Thirty minutes after the stimulus, the vasculature was perfusion-fixed (1% paraformaldehyde in 50 mM citrate buffer, pH 3.5) for 2 min. Ears were removed, blotted dry, and weighed. Evans blue was extracted from the ears with formamide and measured with a spectrophotometer at 610 nm.

We found vessels in the position of capillaries in the skin of K14-Ang1 mice were abnormally large and had phenotypic properties of venules, including strong immunoreactivity for P-selectin and von Willebrand factor. By comparison, in wild type mice the immunoreactivity of these proteins was largely restricted to venules. The microvasculature of ear skin was not leaky in either group of mice under baseline conditions (6.7± 1.7 ng of Evans blue per mg wet tissue weight in K14-Ang1 mice compared to 6.4± 1.6 ng/mg in wild-type mice), but was significantly less leaky in K14-Ang1 mice compared to control after treatment with mustard oil (5.9 ± 1.8 ng/mg in K14-Ang1 vs 18.1 ± 3.9 ng/mg in wild-type mice) - See Figure 2, top panel. Vessels in K14-Ang1 mice were also resistant to leakage after treatment with serotonin - See Figure 2, bottom panel. Although overexpression of Ang1 induced some of the phenotypic features of venules, skin vessels in K14-Ang1 mice are more resistant to leakage induced by inflammatory stimuli.

In summary, we found that the inflammatory mediator mustard oil induced plasma leakage in the ear skin of wild-type mice. However, contrary to our expectations, we found that mustard oil did not induce significant leakage in K14-Ang1 transgenic mice (Figure 2). Furthermore, we found that another inflammatory mediator, serotonin, also failed to induce significant leakage in K14-Ang1 mice (Figure 2).

### Example 2:

In another set of experiments, we found that skin vessels in mice that overexpress VEGF have increased baseline leakage. The vessels in these mice showed further increases in leakage after stimulation with mustard oil. However, unexpectedly, the high baseline leakage in K14-VEGF transgenic mice was reduced to normal values in double transgenic K14-VEGF/Ang1 mice (Figure 3). In addition, mustard oil did not induce significant leakage in double transgenic K14-VEGF/Ang1 mice (Figure 3). These experiments establish that transgenic overexpression of Ang1 results in vessels that are resistant to leakage induced by inflammatory mediators (mustard oil, serotonin) and by transgenic overexpression of VEGF.

To determine whether acute administration of Ang1 can also reduce inflammation-associated leakage, we used an adenovirus encoding for angiopoietin-1* (adeno-Ang1*) to produce high systemic levels in otherwise normal mice. Ang1* has been shown to be a potent agonist for the TIE2 receptor. Plasma leakage was tested in ear skin of mice given this virus by iv injection. Control mice received iv injection of adenovirus encoding for a non-active protein (green fluorescent protein - GFP). At 6 days after administration of adenovirus, the amount of plasma leakage induced by mustard oil was significantly less in mice given adeno-Ang1* than in those given adeno-GFP (Figure 4). This experiment establishes that administration of Ang1 to wild-type mice can result in vessels that are resistant to inflammation-induced leak.

### Example 3:

As stated *supra*, transgenic overexpression of VEGF led to the formation of abnormally leaky vessels, but the leakiness could be normalized by simultaneous transgenic overexpression of Ang1. To determine whether pre-administration of Ad-Ang1 could block leak induced by VEGF in the adult setting, we injected VEGF protein intradermally into the ears of mice that we had previously injected intravenously with Evans blue dye. Dye extravasation resulted, and this extravasation could be blocked by co-administration of a VEGF inhibitor (Fig. 5). Pre-administration of Ad-Ang1 but not Ad-GFP also blocked the leakage induced by VEGF (Fig. 5), demonstrating that short-term delivery of Ang1 could make adult vessels resistant to vascular leak induced by either an inflammatory agent or VEGF.

Evans Blue assays were performed as recently described (Thurston, G. *et al., Science* **286,** 2511-2514 (1999); Suri, C. *et al., Science* **282,** 468-471 (1998); Thurston, G., Baluk, P., Hirata, A. & McDonald, D.M., *Am J Physiol* **271,** H2547-2562 (1996)). In addition, the modified Miles assay in adult mice was performed by injecting Evans blue, followed one minute later by an intradermal injection of VEGF (400 nanograms in 8 microliters) into the dorsal aspect of the middle region of the ear; ears were harvested thirty minutes after injection and processed as in other Evans blue assays. To test the effects of Ang1 in the modified Miles assays and controls depicted in Fig. 5, the mice were injected with 109 PFU of Ad-Ang1 or Ad-GFP 1 to 5 days prior to the assay.

The above studies demonstrate that acute administration of Ang1 protects the adult vasculature against leak induced by a variety of challenges, as was the case with Ang1 transgenically over-expressed during vessel development.

It has long been appreciated that VEGF can rapidly increase vascular permeability in the adult, and that VEGF-induced plasma leakage may contribute to a variety of disease processes. Our data demonstrate, for the first time, that this potentially adverse effect of VEGF can be blocked by acute administration of Ang1.

The present invention is not to be limited in scope by the specific embodiments described herein. Indeed, various modifications of the invention in addition to those described herein will become apparent to those skilled in the art from the foregoing description and accompanying figures.

## Claims

1. Use of a TIE2 receptor activator in the manufacture of a medicament for use in a method of decreasing or inhibiting plasma leakage in a mammal.

2. Use according to claim 1, wherein the mammal is a human.

3. Use according to claim 1 or 2, wherein the TIE2 receptor activator is Ang1, or a polypeptide comprising a fragment or derivative thereof capable of activating the TIE2 receptor.

4. Use according to claim I or 2, wherein the TIE2 receptor activator is an activating antibody, or a polypeptide comprising a fragment or derivative thereof capable of activating the TIE2 receptor.

5. Use according to claim I or 2, wherein the TIE2 receptor activator is a small molecule capable of activating the TIE2 receptor.

6. Use of an anti-Ang-2 neutralising antibody in the manufacture of a medicament for use in a method of decreasing or inhibiting plasma leakage in a mammal.

7. Use according to claim 6, wherein the mammal is a human.

8. Use according to claim 6 or 7, wherein the anti-Ang-2 neutralising antibody is a monoclonal antibody.

9. Use according to claim 8 wherein the monoclonal antibody is a wholly human monoclonal antibody.

10. Use according to claim 6 or 7, wherein the anti-Ang-2-neutralising antibody is a polyclonal antiserum.

## Patentansprüche

1. Verwendung eines TIE2-Rezeptor-Aktivators für die Herstellung eines Medikaments zur Verwendung in einem Verfahren zur Verringerung oder Verhinderung des Plasmaaustritts in einem Säuger.

2. Verwendung nach Anspruch 1, wobei der Säuger ein Mensch ist.

3. Verwendung nach Anspruch 1 oder 2, wobei der TIE2-Rezeptor-Aktivator Ang1 oder ein Polypeptid ist, umfassend ein Fragment oder Derivat davon, das den TIE2-Rezeptor aktivieren kann.

4. Verwendung nach Anspruch 1 oder 2, wobei der TIE2-Rezeptor-Aktivator ein aktivierender Antikörper oder ein Polypeptid ist, umfassend ein Fragment oder Derivat davon, das den TIE2-Rezeptor aktivieren kann.

5. Verwendung nach Anspruch 1 oder 2, wobei der TIE2-Rezeptor-Aktivator ein kleines Molekül ist, das den TIE2-Aktivator aktivieren kann.

6. Verwendung eines neutralisierenden anti-Ang-2-Antikörpers für die Herstellung eines Medikaments zur Verwendung in einem Verfahren zur Verhinderung oder Hemmung des Plasmaaustritts in einem Säuger.

7. Verwendung nach Anspruch 6, wobei der Säuger ein Mensch ist.

8. Verwendung nach Anspruch 6 oder 7, wobei der neutralisierende anti-Ang-2-Antikörper ein monoclonaler Antikörper ist.

9. Verwendung nach Anspruch 8, wobei der monoclonale Antikörper ein gänzlich menschlicher monoclonaler Antikörper ist.

10. Verwendung nach Anspruch 6 oder 7, wobei der neutralisierende anti-Ang-2-Antikörper ein polyclonales Antiserum ist.

## Revendications

1. Utilisation d'un activateur du récepteur TIE2 dans la préparation d'un médicament destiné à être utilisé dans un procédé de diminution ou d'inhibition de la perte de plasma chez un mammifère.

2. Utilisation selon la revendication 1, dans laquelle le mammifère est un humain.

3. Utilisation selon la revendication 1 ou 2, dans laquelle l'activateur du récepteur TIE2 est Ang-1, ou un polypeptide comprenant un fragment ou un dérivé de celui-ci capable d'activer le récepteur TIE2.

4. Utilisation selon la revendication 1 ou 2, dans laquelle l'activateur du récepteur TIE2 est un anticorps activant, ou un polypeptide comprenant un fragment ou un dérivé de celui-ci capable d'activer le récepteur TIE2.

5. Utilisation selon la revendication 1 ou 2, dans laquelle l'activateur du récepteur TIE2 est une petite molécule capable d'activer le récepteur TIE2.

6. Utilisation d'un anticorps neutralisant anti-Ang-2 dans la préparation d'un médicament destiné à être utilisé dans un procédé de diminution ou d'inhibition de la perte de plasma chez un mammifère.

7. Utilisation selon la revendication 6, dans laquelle le mammifère est un humain.

8. Utilisation selon la revendication 6 ou 7, dans laquelle l'anticorps neutralisant anti-Ang-2 est un anticorps monoclonal.

9. Utilisation selon la revendication 8, dans laquelle l'anticorps monoclonal est un anticorps monoclonal entièrement humain.

10. Utilisation selon la revendication 6 ou 7, dans laquelle l'anticorps neutralisant anti-Ang-2 est un antisérum polyclonal.
